# EUROPEAN PATENT SPECIFICATION

(11) **EP 3 747 357 B1**
(45) Date of publication and mention of the grant of the patent: **15.02.2023**
(21) Application number: 18904350.8
(22) Date of filing: 31.01.2018
(51) Int. Cl.: A61B 5/346, G16H 50/70, A61B 5/35

(54) **MEDICAL SYSTEM AND METHOD FOR REMOTE PATIENT MONITORING**
MEDIZINISCHES SYSTEM UND VERFAHREN ZUR FERNÜBERWACHUNG EINES PATIENTEN
SYSTÈME ET MÉTHODE MÉDICALES DE SURVEILLANCE DE PATIENT À DISTANCE

(43) Date of publication of application: 09.12.2020
(73) Proprietor: ZAKRYTOE AKTIONERNOE OBSCHESTVO "EC-LEASING", Moscow 117405 (RU)
(72) Inventor: SHMID, Alexander Viktorovich, Moscow 125310 (RU); BEREZIN, Andrey Alexandrovich, Moscow 125368 (RU); NOVOPASHIN, Maxim Alexandrovich, Ivanovskaya obl. Vichuga 155335 (RU)
(74) Representative: Gevers & Orès
(86) International application number: PCT/RU2018/000054
(87) International publication number: WO 2019/151888

(56) References cited:
- WO-A1-2017/178643
- RU-C1- 2 383 295
- RU-C2- 2 428 104
- US-A1- 2004 230 105
- US-A1- 2006 079 795
- US-A1- 2009 192 394
- US-A1- 2010 049 069
- US-A1- 2017 347 964
- US-B2- 7 485 095
- PAOLETTI M ET AL: "Discovering dangerous patterns in long-term ambulatory ECG recordings using a fast QRS detection algorithm and explorative data analysis", COMPUTER METHODS AND PROGRAMS IN BIOMEDICINE, ELSEVIER, AMSTERDAM, NL, vol. 82, no. 1, 1 April 2006 (2006-04-01), pages 20-30, XP024895775, ISSN: 0169-2607, DOI: 10.1016/J.CMPB.2006.01.005 [retrieved on 2006-04-01]
- VORONTSOV K.V.: "Lectures on clustering algorithms and multidimensional scaling", Machine Learning, 19, 24 June 2010 (2010-06-24), XP055628065, Retrieved from the Internet: URL:http://www.machinelearning.ru/wiki/ima ges/c/ca/Voron-ML-Clustering.pdf

## Description

### Technical Field

The present invention relates to medicine and, more specifically, to a medical system for remote monitoring, analysis and prognosis of the patient's condition by series of at least first-lead electrocardiograms (ECG) recorded for each of the observed patients, and to a computer-assisted method for monitoring/analysis and prognosis of the patient's condition by first-lead electrocardiogram series (ECGs).

The invention can be used for improving the accuracy of assessment of the patient's condition by automatically providing more information (Big Data) to ECG specialists and ECG transcriptionists and for evaluating the effectiveness of medication treatment based on a cardiogram and/or pulse wave.

### Background of the Invention

Cardiovascular diseases remain the leading cause of death worldwide, and coronary heart disease (CHD) contributes most to the overall picture of mortality. In Europe, mortality from cardiovascular diseases in 2013 was about 49%. In Russia in 2009, the CHD accounted for 28.9% of all deaths.

This fact causes the urgency of the problem of predicting the condition in patients with chronic coronary heart disease (CCHD) in order to choose the proper therapeutic approach. The amount of therapy required to a particular CHD patient should be primarily determined from individual risk which can vary significantly.

The high-priority general scientific application is the prognosis, which, using mathematical models and methods, would enable determining the tolerance level of the human body to effects of various factors in order to specify the predisposition to a particular disease, and if the predisposition has already arisen, predicting the specifics of its future progression and the outcome.

To successfully solve this problem, it is necessary to provide an algorithm available to any practitioner, which would enable dividing patients into risk groups and selecting the optimal treatment approach for a specific patient from the prognosis viewpoint. To date, there is no uniform method for calculating the risk of death and cardiovascular events.

A method for predicting the progression of coronary heart disease is disclosed in patent RU 2391044 (published 10.06.2010). The method involves clinical and electrocardiographic examinations, Holter monitoring, echocardiography, bicycle ergometry, heart rate variability analysis, signal-averaged electrocardiography, determination of QT interval variance. The method uses a staged examination of patients, where the first stage comprises clinical examination, the second stage - Holter ECG monitoring, the third stage - echocardiography, the fourth stage - stress test, and the fifth stage comprises analysis of the average ECG heart rate variability signal. At each stage, methods of multivariate statistical analysis distinguish high-risk predictors of occurrence of the events that are lifethreatening for patients with chronic coronary insufficiency. Unfavorable prognosis for coronary heart disease is predicted if the first stage identifies: presence of angina pains, irregular heartbeat, syncope condition; arterial hypertension, diabetes mellitus, smoking, physical inactivity, hyperlipidemia, psycho-emotional stress, presence of criteria for cicatricial changes and evidences of ischemia indicants, presence of ventricular rhythm disturbances, including high gradations, lengthening of QT interval in analysis of QT dispersion at resting electrocardiography. Unfavorable identifiers at the second stage include: daily duration of all ischemic episodes more than 60 minutes; presence of painless episodes of ischemia; maximum depth of ST segment depression of more than 3 mm; the number of heart contractions at the beginning of pain episodes less than 110 per minute; the number of heart contractions at the beginning of painless episodes less than 100 per minute; presence of extrasystole of high IV, V gradations, runs of ventricular tachycardia; presence of ventricular extrasystole. Unfavorable identifiers at the third stage include: finite diastolic size of more than 6.0 cm, ejection fraction of less than 40%, presence of hypoand akinesia zones, evidences of aneurysm. Unfavorable identifiers at the fourth stage include: decrease in airway tolerance to physical activity of less than 210 conventional units, maximum heart rate at the beginning of ischemic episodes less than 100 per minute; presence of ventricular arrhythmias associated with transient episodes of ischemia, the number of leads with ST segment depression more than 6. Unfavorable identifiers at the fifth stage include: presence of late ventricular potentials, SDNN values less than 21 ms, BB50 less than 10 beats/min, RMSDD less than 15 ms, LF/HF more than 1.7 conventional units, triangular index less than 15.

Based on the results of the phased examination, the probability of a favorable or unfavorable CHD progression is calculated using the length of vectors of CHD course variants and, accordingly, the probability of a high or low risk of death of patients is predicted.

This method is unable to provide effective diagnosis of patients outside the healthcare center when determining the patient's condition and rendering aid to both the patient in express assessment of his current condition and the clinician in monitoring the patient's treatment outside the healthcare center.

RU 2615721 B2 discloses a device for cardiographic monitoring of the patient's condition, comprising a monitor, an interface, at least one electric cardio signal sensor (ECG electrode) mounted on a patient and designed to pick up electric cardio signals from the patient's body, connected by an output to input of a primary signal processing unit, the other input of this unit being connected to output of the time sampling unit, and output of the primary signal processing unit is connected to a channel switching unit. Outputs of the channel switching unit are connected to a discrete Fourier transform unit which outputs values of the amplitude, frequency and phase of harmonics of the signal under study, and to a patient data input unit. Harmonics are processed in a cardiogram register that stores and outputs harmonic amplitudes of the signal under study for required amount of time. The harmonic amplitudes are input into a cardiogram image identifier, which compares the image received from the ECG electrode, taking into account the confidence intervals and a certain degree of reliability, with images from the cardiogram image base. Output of the identifier is connected to input of a unit for recording the states and analyzing their dynamics, where a patient's disease diagnosis is formed on the basis of data of the cardiogram images from all ECG electrodes by comparing the set of cardiogram images from ECG electrodes with a disease diagnosis characterizing set from the cardiological diagnosis base, taking into account the confidence intervals and a certain degree of reliability. The same unit determines the diagnosis reliability level, the diagnosis dynamics depending on the previous examination of the patient, and the diagnosis determination time. The data is displayed on the monitor, transferred to the interface for storage and study at other technical means and to the patient data input unit, where they are stored in the respective patient's archives.

The device is unable to provide effective diagnosis of patients outside the healthcare center when determining the patient's condition and assisting both the patient in express assessment of his current condition and the clinician in monitoring the patient's treatment outside the healthcare center.

Most closely related to the present invention is CardioQVARK portable system (O. Yu. Pidanov, "The first experience of personal ECG monitoring in patients after thoracoscopic ablation of the left atrium," Vestnik Sovremennoy klinicheskoy meditsiny, 2017, v.10, issue 6, p.p. 24-30). The system serves as a tool for cardiovascular system monitoring in patients after thoracoscopic ablation due to atrial fibrillation. 5 minute ECG recordings were taken with CardioQVARK portable monitor in hospital patients from 1st to 5th day after the surgery, and data was transmitted by mobile Internet via cloud service to CardioQvark Doctor application. The hospital stage has demonstrated convenience and ease of use of the personal CardioQVARK monitor when monitoring patients in the period after surgical treatment for atrial fibrillation. Remote monitoring by CardioQVARK allows continuous observation of patients.

Use of iPad CardioQVARK Doctor application enables gathering of a research base, which is convenient for quick access, and quick analysis of the cardiogram QT, PQ interval for finding antiarrhythmic medication dose.

Test investigation comprised monitoring ECG patients daily for 2 months when they were out of the hospital. 64 ECG recordings with a total duration of 262 minutes were obtained. Of the obtained records, 61 (95.3%) were suitable for analysis. The investigation provided for recording ECG daily in the morning, and recording and transmitting data when a sensation of irregular heartbeat occurred i.e. event monitoring.

The monograph states that the use of CardioQVARK device precludes the complete omission of using bedside monitors, ECG devices, Holter monitors, etc.

The advantage of long-term and, even better, permanent electrocardiographic (ECG) monitoring cannot be doubted. However, in real clinical practice, the use of monitors is extremely limited due to their high cost.

When an arrhythmia attack occurs, its recording may be impossible simply because the attack can stop on its own before the patient reaches the ECG office or the ambulance arrives. Under such conditions, it becomes extremely attractive to monitor cardiac rhythm disturbances with the ability to transmit the results to a specialist at a distance using current communication technologies.

The above system is unable to provide prognosis of the observed patient's condition.

Systems for remote ECG monitoring and for predicting patient outcome using clustering methods are also known from WO-2017/178643-A1, US-2017/347964-A1, US-2004/230105-A1, US-2010/049069-A1, US-2006/079795-A1, and PAOLETTI M ET AL: "Discovering dangerous patterns in long-term ambulatory ECG recordings using a fast QRS detection algorithm and explorative data analysis",COMPUTER METHODS AND PROGRAMS IN BIOMEDICINE, ELSEVIER, AMSTERDAM, NL, vol. 82, no. 1, April 2006, pages 20-30.

### SUMMARY OF THE INVENTION

The object of the present invention is to provide a medical system for remote analysis of cardiac data obtained from at least one patient outside the healthcare center, analysis and prognosis of the patient's condition based on series of at least first-lead electrocardiograms (ECG) recorded for each of the observed patients, which can quickly and/or reliably overcome the aforementioned problems of the prior art.

Another object of the present invention is to provide a computer-assisted method for monitoring, analysis and prognosis of the patient's condition based on series of at least first-lead cardiac electrocardiograms (ECGs) and other cardiological signals synchronized with ECG, using this medical system, which can enable efficient analysis, diagnosis and prognosis of the patient's condition and render aid to the clinician in treatment planning.

The advantage provided by the present invention comprises the provision of effective diagnosis of patients outside the healthcare center when determining the patient's condition and assistance to both the patient in express assessment of his current condition and the clinician in monitoring the progression of patient's treatment outside the healthcare center, including the assessment of cardiotoxicity in the use of medications, not only cardiac ones.

Therefore, according to a first aspect of the present invention the object is attained by providing a medical system for remote monitoring, analysis and prognosis of the patient's condition by series of at least first-lead electrocardiograms (ECGs) recorded for each patient from a plurality of observed patients, comprising:
a unit for gathering in a database of said medical system for each of the observed patients a series of his electrocardiograms (ECGs) marked with patient identifiers, generated by adding each newly recorded patient's ECG marked with said identifiers to the patient's ECG series gathered previously throughout the entire observation time;
a unit for extracting on doctor's request from the gathered database an ECG subset of a target group of patients, which are combined in said system into the target group in accordance with restrictions specified in the doctor's request and at least one of said identifiers in accordance with the examination purpose;
a unit for clustering the extracted ECG subset, which sequentially combines, for at least one target group of patients combined by at least one feature, all the ECGs extracted to the subset to at least one cluster based on the feature of similarity of their forms using a clustering method;
a unit for determining an ECG form similarity measure in the space of ECG form features, which associates each ECG from the database of the medical system with a discretized ECG (DECG), which is a point having coordinates in at least one space of ECG form features, chosen by the system operator, wherein coordinates of the point in said space of ECG form features carry information about the original ECG form, and the ECG form similarity measure between any ECG pairs is specified as the distance between their corresponding pairs of DECG points in this space;
a unit for generating STANDARDS, which generates, for each cluster formed by the unit (3), a corresponding STANDARD by forming a set of DECG points corresponding to the cluster ECGs, and calculates characteristics of the formed DECG set, including: coordinates of the center of gravity of the formed DECG set, radius of the sphere or center of the polygon at the center of gravity of the DECG set, including all the DECG sets;
a unit for assigning, on doctor's request, at least one STANDARD in accordance with the examination purpose from a plurality of generated STANDARDS for subsequent assessment of the patient's condition relative to the condition corresponding to the at least one STANDARD assigned by the doctor;
a unit for assessing the patient's condition by the patient's ECG series, which assesses variation in the observed patient's condition over time by temporal variation in the distances between each newly received DECGs in the patient's DECG series and the assigned at least one STANDARD;
a unit for predicting the observed patient's condition, which predicts the most probable future condition of the patient on the basis of "history of observations" comprising recorded variations of past observed patient's conditions over time relative to the assigned at least one STANDARD;
a unit for imaging the patient's condition, which displays the dynamics of variation in distances of the patient's newly received ECGs as a displacement of cardiogram geometric paths between the assigned STANDARDS.

Preferably, the unit for predicting the observed patient's condition outputs a prognosis of the observed patient's condition in future periods of time, starting from the last instant of recording the patient's ECG, based on the type of variation in the observed patient's ECG path relative to the center of gravity of the assigned at least one STANDARD.

Preferably, the patient identifiers contain two sets of features, a first set of features defining the patient's personal data code, and a second set of features comprising parameters of the outpatient's card selected from the group consisting of features of gender, age, diagnosis, social status, blood type, profession, belonging to social groups, education, genotype, with/without pathology, taking/not taking medications.

Preferably, the form similarity measure between any ECG pairs is specified as the distance between their corresponding pairs of DECG points, calculated by formulas for calculating the distance between points in at least one selected space: Euclidean space, Riemannian space, Lobachevsky space, Hilbert space.

Preferably, the clustering method is selected from the group consisting of: K-means, K-medians, C-means, EM, FOREL, Kohonen neural network, graph methods, including single link methods, complete link methods, Ward method, average link methods, and weighted average link methods.

Preferably, the clustering unit checks, for the next analyzed ECG related to the extracted subset, the measure of similarity of its form with forms of all ECGs in each of the already formed clusters, and
if the analyzed ECG according to the predetermined form similarity measure matches at least one of the ECGs of one already formed cluster, then the ECG corresponds to this cluster and falls into this cluster;
if the ECG corresponds to more than one cluster, the previously formed clusters, to which the analyzed ECG corresponds, are merged into a single cluster;
if the ECG does not correspond to any of the previously formed clusters, a new cluster containing this single ECG is formed.

Preferably, in long-term ECGs a finite time interval from 1 to 5 minutes is selected, and DECG is determined in this time interval for further calculation of the similarity measure.

Preferably, in long-term ECGs more than one finite interval is selected; DECG is determined in these time intervals for further calculation of the similarity measure, and a path of patient's condition variation during the ECG recording procedure is constructed based on the similarity measure.

Preferably, the space of features is normalized by reducing the recorded ECGs to one fixed length using an interpolation method.

According to a second aspect of the invention the object of the invention is attained by providing a computer-assisted method for remote monitoring, analysis and prognosis of the patient's condition using a medical system according to claim 1, comprising:
gathering, for each patient from a plurality of observed patients, a series of patient's at least first-lead electrocardiograms (ECGs) marked with patient's identifiers in a database of said medical system, generated by adding each newly recorded patient's ECG marked with said identifiers to the patient's ECG series gathered previously throughout the entire observation period;
extracting, on doctor's request, from the gathered database an ECG subset of a target group of patients, which are combined into a target group in accordance with restrictions specified in the doctor's request and at least one of said identifiers in accordance with the examination purpose;
clustering the extracted ECG subset for the at least one target group of patients combined by at least one feature, for which purpose all the ECGs extracted to the subset are sequentially combined into at least one cluster by the feature of similarity between their forms using one of clustering methods;
determining a similarity measure of ECG forms in the space of ECG form features, for which purpose each ECG from the medical system database is associated with a discretized ECG (DECG), which is a point having coordinates in at least one space of ECG form features chosen by the system operator, and coordinates of the point in said space of ECG form features carry information about the original ECG form, and the measure of ECG form similarity between any ECG pairs is specified as the distance between their corresponding pairs of DECG points in this space;
generating a respective STANDARD for each cluster by forming a set of DECG points corresponding to the cluster ECGs and calculating characteristics of the formed DECG set, including: coordinates of the center of gravity of the formed DECG set, radius of the sphere or center of the polygon at the center of gravity of the DECG set, including all DECGs sets;
assigning at least one STANDARD from a set of generated standards for subsequent assessment of the patient's condition in accordance with the examination purpose specified by the doctor and comparing the patient's condition with the condition corresponding to the assigned at least one STANDARD;
assessing variation of the observed patient's condition over time by temporal variation in distances between each of the newly received DECGs in the patient's DECG series and the assigned at least one STANDARD;
predicting the observed patient's condition based on "observation history" comprising recorded variations in past observed patient's conditions over time relative to the assigned at least one STANDARD;
imaging the patient's condition as the dynamics of variation in the distances of the newly received patient ECGs at a displacement of geometric paths between the assigned STANDARDS.

Preferably, the observed patient's condition is predicted for future time periods, starting from the last instant of recording the patient's ECG, based on the type of variation in the observed patient's ECG path relative to the center of gravity of the assigned at least one STANDARD.

Preferably, the patient identifiers are selected from two sets of features, a first set of features defining the patient's personal data code, and a second set of features comprising parameters of the outpatient's card selected from the set consisting of features of gender, age, diagnosis, social status, blood type, profession, belonging to social groups, education, genotype, with/without pathology, taking/not taking medications.

Preferably, said recording of the ECG series is carried out according to a predetermined examination method, which provides for recording ECGs at specified instants and/or upon the occurrence of predetermined events associated with the patient's health status, patient's daily routine, taking medications or medical procedures.

Preferably, the form similarity measure between any ECG pairs is specified as the distance between their corresponding pairs of DECG points, calculated by formulas for calculating the distance between points in at least one space: Euclidean space, Riemannian space, Lobachevsky space, Hilbert space.

Preferably, the clustering method is selected from the group consisting of: K-means, K-medians, C-means, EM, FOREL, Kohonen neural network, graph methods, including single link methods, complete link methods, Ward method, average link methods, and weighted average link methods.

Preferably, the next analyzed ECG related to the extracted subset is clustered, for which purpose the similarity measure of its form with forms of all ECGs in each of the already formed clusters is checked, and
if the analyzed ECG matches, by the predetermined form similarity measure, at least one of ECGs of the already formed one cluster, the ECG is considered as corresponding to this cluster and falling into this cluster;
if the ECG corresponds to more than one cluster, the previously formed clusters, to which the analyzed ECG corresponds, are merged into a single cluster;
if the ECG does not correspond to any of the previously formed clusters, a new cluster containing this single ECG is formed.

Preferably, the "with/without pathology" feature includes the presence or absence of pathology in the patient's diagnosis according to the International Classification of Diagnoses (ICD), confirmed by the doctor.

Preferably, the method further comprises using electrocardiograms selected from the set consisting of Eindhoven electrocardiogram, pulse wave cardiogram (photoplethysmogram), oxyhemogram, respiration card, echocardiogram, seismocardiogram and cardiovascular system signals synchronized with ECG.

### Brief Description of the Drawings

The invention is further explained in the description of the preferred embodiment with reference to the accompanying drawings, in which:
Fig. 1 shows a schematic diagram of a medical system for remote monitoring, analysis and prognosis of the patient's condition, according to the invention;
Fig. 2 shows a scheme of forming STANDARDS;
Fig. 3 shows a diagram of a geometric path of newly arrived patient's ECGs between assigned STANDARDS;

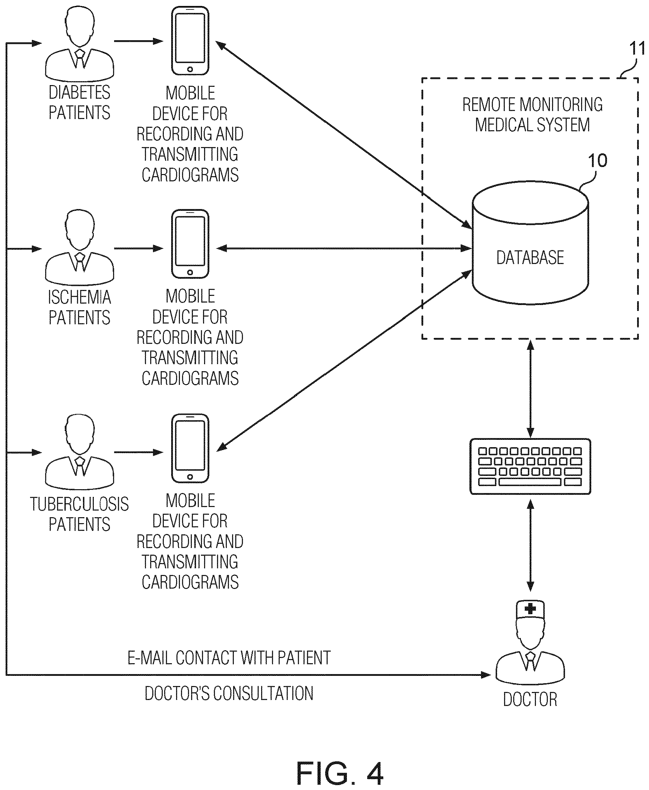

Fig. 4 is a schematic diagram of gathering patient's ECGs by transmitting data from a cell phone, with data transmission over the mobile operator Internet.

### Theoretical Basis of the Present Method

In 1954, Fermi, Pasta, and Ulam made an attempt to demonstrate numerically that a nonlinear system inevitably transits from a state of single-mode excitation to a state with equally distributed energy.

The inventors proposed and mathematically described a variant of the Fermi-Pasta-Ulam recurrence model (FPU auto recurrence) and hypothesized an adequate description of the heart's electrical dynamics within the detected FPU auto recurrence phenomenon. The dynamics of the FPU auto recurrence making appropriate electrical dynamics of the normal functioning of the heart in the form of an electrocardiogram (ECG) was obtained by a computer model study (see A.V. Shmid, A.A. Berezin, M.A. Novopashin, Fermi-Pasta-Ulama auto recurrence in the description of the electrical activity of the heart, Journal Medical Hypothesis, 101 (2017) pp. 17-22).

The model studies have revealed a hypothetical basis of coronary heart disease in the form of increasing the energy of high-frequency harmonics spectrum of the FPU auto recurrence by reducing the energy of low-frequency harmonic spectrum of the auto recurrence, which ultimately leads to a sharp decrease in myocardial contractility.

The hypothesis was tested on more than 20,000 ECGs of both healthy individuals and patients with cardiovascular disease that have been studied. The studies revealed that the dynamics of electrical activity of a normally functioning heart can be interpreted by the FPU auto recurrence phenomenon, and coronary heart disease is a violation of the energy ratio between the low and high frequency harmonics of the FPU auto recurrence.

To simulate the ECG, it was taken into account that the heart functions in a self-oscillating mode, which implies the presence of a similar principle of dynamics in the FPU recurrence structure, used for simulating cardiac activity.

The mathematical simulation shows that electrical activity of the heart is the FPU auto recurrence, first formulated by the inventors, which has an individual nature and contains in its structure a picture of the physiological state of the heart and pathological processes whose patterns periodically appear in the Fourier spectra of auto recurrences.

Auto recurrences of Fourier spectra of healthy individuals ECGs comprise both a low-frequency part in the range of 1-5 Hz, corresponding to the canonical FPU recurrence, and a high-frequency part in the range of 20-35 Hz, corresponding to the high-frequency FPU recurrence. Therefore, ECG is a biological example of a full FPU auto recurrence.

Based on the simulation results, it was suggested that the set of frequency patterns obtained from different cardiograms of one patient or several patients does not have a chaotic distribution, and therefore several sets of frequency patterns can be distinguished by some measure of proximity.

This means that clustering algorithms can be applied to the normalized space of ECG frequency patterns, and stable frequency patterns of conditions of both a particular patient and a group of patients selected for a common feature can be obtained.

### Description of Preferred Embodiment

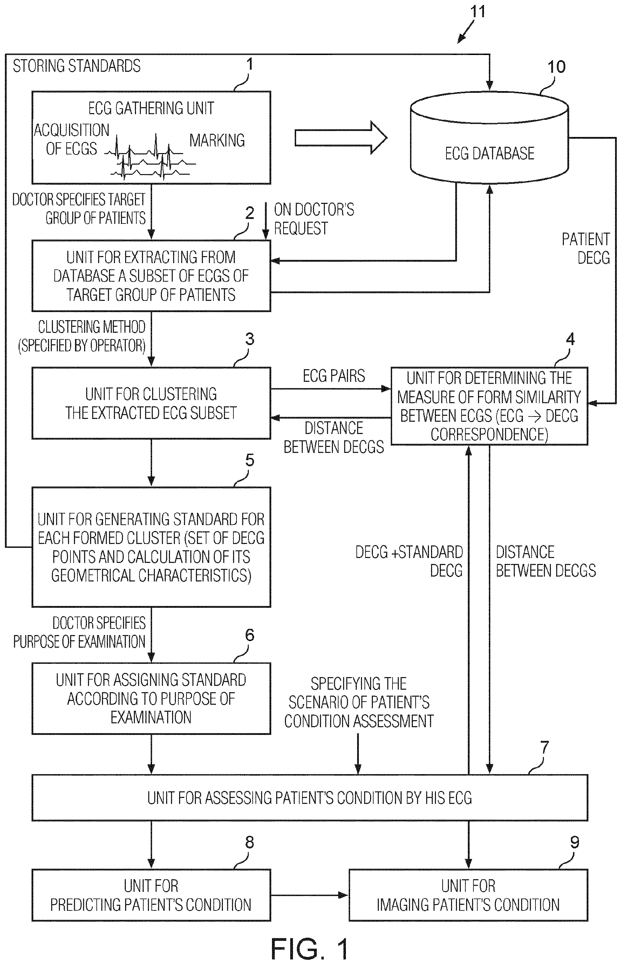

According to the invention, a medical system for remote monitoring, analysis and prognosis of the patient's condition by series of at least first-lead electrocardiograms (ECG) recorded for each patient from a plurality of observed patients comprises a unit 1 (Fig. 1) for gathering a series of patient's electrocardiograms (ECGs) in a database of said medical system for each of the plurality of observed patients. The electrocardiograms are marked with patient identifiers. Patient identifiers are selected from the set consisting of features of gender, age, diagnosis, social status, blood type, profession, belonging to social groups, education, genotype, with/without pathology, taking/not taking medications.

The series of electrocardiograms (ECGs) is generated by adding each new recorded patient's ECG marked with identifiers to the patient's ECG series gathered previously throughout the entire observation time.

The medical system further comprises a unit 2 for extracting, on doctor's request, from the gathered database a subset of ECGs belonging to a target group of patients, which are combined in the system into the target group in accordance with restrictions specified in the doctor's request and at least one of the identifiers in accordance with the examination purpose.

Each cardiogram received from the database is provided with a patient code and, if necessary, a medical identifier, i.e. it specifies who is being studied and what is being studied, for example, cardiograms of young individuals under the age of 30 who are constantly taking analgesics are being studied.

The medical system comprises a unit 3 for clustering the extracted subset of ECGs. The unit 3 sequentially combines, for at least one target group of patients combined by at least one identifier, all the ECGs extracted to the subset to at least one cluster A1, A2, A3, A4, A5 based on the similarity between their forms using a clustering method.

A conventional clustering method is used, which is selected from the set consisting of: K-means, K-medians, C-means, EM, FOREL, Kohonen neural network, graph methods, including single link methods, complete link methods, Ward method, average link methods, and weighted average link methods.

A unit 4 for determining a similarity measure of ECG forms determines the ECG form similarity measure in the space of ECG form features. From the clustering unit 3, the electrocardiograms are sequentially transferred in pairs to the unit 4 for determining the ECG form similarity measure to get a response i.e. the distance (similarity measure) between DECGs of the transferred ECG pair.

The unit 4 associates each ECG received from the medical system database and transferred from the clustering unit 3 with a discretized ECG (DECG), which is a point having coordinates in at least one space of ECG form features chosen by the system operator. Values of coordinates of the point in this ECG form feature space carry information about the form of the original ECG, and the form similarity measure between any ECG pairs is specified as the distance between their corresponding pairs of DECG points in this space. The space of similarity features is specified by the operator.

The clustering unit 3 checks, for the next analyzed ECG related to the extracted subset, the measure of its form similarity with forms of all ECGs in each of the already formed clusters A1 to A5.

If according to the predetermined form similarity measure the analyzed ECG matches at least one of the ECGs of one already formed cluster, then the ECG corresponds to this cluster and falls into this cluster. If the ECG corresponds to more than one cluster, then the previously formed clusters, which correspond to the analyzed ECG, are merged into a single cluster, for example, A3 is merged with A4. If the ECG does not correspond to any of the previously formed clusters, a new cluster A5 containing this single ECG is formed.

Furthermore, the measure of form similarity between any ECG pairs is specified as the distance between their corresponding pairs of DECG points, calculated by formulas for calculating the distance between points in at least one selected space: Euclidean space, Riemann space, Lobachevsky space, Hilbert space.

In long-term ECGs, a finite interval of 1 to 5 minutes is chosen, and DECG is determined in this interval for further calculation of the similarity measure.

In long-term ECGs, more than one finite interval is chosen, and the DECG is determined in these intervals for further calculation of the similarity measure, and the path of patient's condition variation during the procedure of recording the long-term ECGs is constructed on the basis of the similarity measure.

The feature space is normalized by reducing the recorded ECGs to one fixed length using interpolation method.

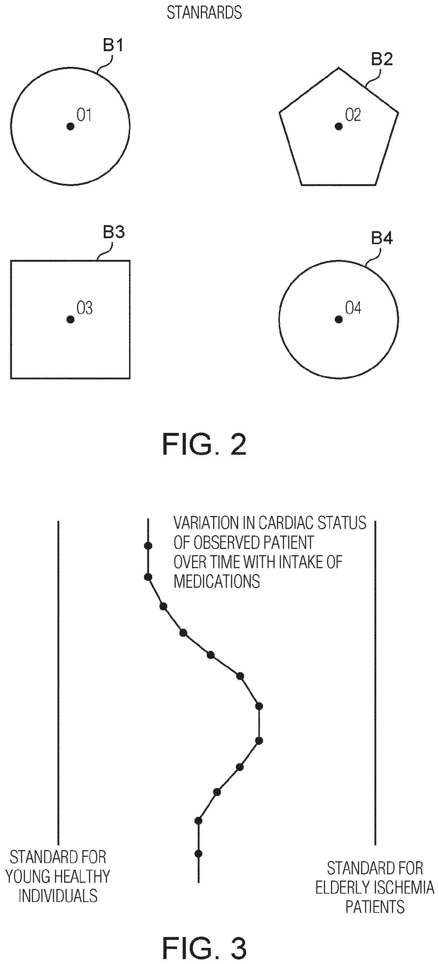

The medical system comprises a unit 5 for generating a respective STANDARD for each cluster A1 to A4 formed by the clustering unit 3 by forming a set of DECG points corresponding to the cluster ECGs, and calculating characteristics of the formed set of DECGs. The characteristics include: coordinates of the center of gravity 01, 02, 03, 04 of the generated DECG set, radius of the sphere or center of the polygon at the center of gravity of the DECG set, which includes all DECG sets. Fig. 2 shows STANDARDS B1, B2, B3 and B4.

The generated STANDARDS can be stores in the ECG database.

An assignment unit 6 assigns STANDARD on the doctor's request of at least STANDARD in accordance with the examination purpose for subsequent assessment of the patient's condition relative to the condition corresponding to the at least one STANDARD assigned by the doctor from the set of formed STANDARDS. Therefore, the assigned STANDARD corresponds to the condition indicated in the examination purpose. Optionally, the stored STANDARD can be received from the ECG database.

Then, a unit 7 for assessing the patient's condition by his ECG series assesses the variation in the observed patient's condition over time by temporal variation in the distance between each of the newly received DECGs in the patient's DECG series and the assigned at least one STANDARD. Since the STANDARD is formed, the doctor or the patient himself can compare the ECG series of one patient's, received from the database, with the STANDARD. Fig. 3 shows a DECG displacement diagram of the observed patient who is taking medications, where the left side shows the STANDARD of young healthy persons, the right side shows the STANDARD of elderly ischemia patients.

The present medical system provides prognosis by a unit 8 for predicting the observed patient's condition, in which the most probable future condition of the patient is predicted based on "observation history" comprising recorded variations of past conditions of the observed patient over time relative to the assigned at least one STANDARD.

The unit 8 for predicting the observed patient's condition outputs a prognosis of the observed patient's condition for future periods of time, starting from the last instant of recording the patient's ECG, based on the type of variation in the ECG path of the observed patient (Fig. 3) relative to the center of gravity of the assigned at least one STANDARD.

An imaging unit 9 displays the patient's condition, for which purpose the dynamics of variation in the distances of the newly received patient ECG is displayed as a displacement of geometric paths between the assigned STANDARDS (Fig. 3).

The medical system can also be applied to a group of patients.

The invention also provides a computer-assisted method for remote monitoring, analysis and prognosis of the patient's condition, which uses the medical system and compares forms of a set of patient's ECGs as carriers of information about the actual patient's health status.

In the present method, the computer first trains, i.e. generates a set of diagnosis STANDARDS, each comprising a multitude of similar in form ECGs of previously observed patients, corresponding to one of the STANDARDS set by the doctor, characterizing the alleged diagnosis.

Further, the trained computer is used to assess the measure of ECG form proximity of a patient with an unknown diagnosis with one of the STANDARDS formed in the course of computer training.

Current condition of the patient is assessed by the measure of proximity of his ECG form to the ECG forms of one or more STANDARDS.

Variation in the patient's condition over time is assessed based on the analysis of his ECG series whether the patient's ECG form similarity measure is approaching or separating from the ECG STANDARD forms.

Prognosis of the patient's condition progression is made on the basis of analysis of the history of variation in the form similarity measure of his ECG series relative to the ECG STANDARD forms.

Measurement of the ECG form similarity measure relies on the ability to represent each of the patient's ECGs as a point (DECG) with coordinates that reflect the ECG form in a selected space of ECG form similarity features. The introduction of the space of ECG form similarity features makes it possible to further operate with geometric terms, in other words, to quantify the ECG form similarity measure as the distance between points (DECG), and consider the diagnosis STANDARD as a set of points, i.e. an area in the space of form similarity features. Therefore, the method enables the transition from a visual informal assessment of the ECG form as the basis of diagnosis to a formal computer-assisted assessment.

Hereinafter are steps of a method for remote monitoring, analysis and prognosis of the patient's condition using first-lead electrocardiogram series (ECG) recorded for one of the observed patients.

To implement the method, it is initially required to gather, for each patient from a plurality of observed patients in the medical system database, a series of at least first-lead electrocardiograms (ECGs) marked with patient identifiers. The series of electrocardiograms (ECGs) is generated by adding each newly recorded ECG of a patient from the observed plurality of patients, marked with identifiers, to the patient's ECG series gathered previously throughout the entire observation time. Thus, the database comprises a multitude of at least first-lead electrocardiograms (ECGs).

The electrocardiograms are marked with patient identifiers. Patient identifiers are selected from the features of sex, age, diagnosis, social status, blood type, profession, belonging to social groups, education, genotype, with/without pathology, taking/not taking medications.

Then, in accordance with doctor's request, the unit 3 extracts a subset of ECGs of the target group of patients from the gathered database. Patients are combined into the target group in accordance with restrictions specified in the doctor's request and at least one of the identifiers pursuant to the examination purpose. For example, cardiograms of young individuals under the age of 30 years who are constantly taking analgesics are studied. Or a doctor may request all the patients taking a set of medications.

Each cardiogram received from the database is provided with a patient code and, if necessary, a medical identifier, i.e. contains information about who is being studied and what is being studied.

Through this process a subset of ECGs of the target patient group is generated.

Then, the unit 3 performs clustering of the extracted ECG subset of the target group of patients combined by at least one identifier, for this purpose all the ECGs extracted to the subset are sequentially combined into at least one cluster on the basis of similarity between their forms using one of clustering methods.

Clustering method is selected from the set consisting of K-means, K-medians, C-means, EM, FOREL, Kohonen neural network, graph methods, including single link methods, complete link methods, Ward method, average link methods, weighted average link method.

The unit 4 for determining ECG form similarity measure determines ECG form similarity measures in the space of ECG form features. Electrocardiograms from the clustering unit are transferred in pairs to the ECG form similarity measure determining unit 4 to receive a response, i.e. the distance (similarity measure) between DECGs of the transferred ECG pair.

The unit 4 associates each ECG received from the medical system database and transferred from the clustering unit 3 a discretized ECG (DECG), which is a point having coordinates in at least one ECG form feature space chosen by the system operator. Values of coordinates of the points in the ECG form feature space carry information about the form of the original ECG, and the measure of similarity between forms of any ECG pairs is specified as the distance between their corresponding pairs of DECG points in this space. The similarity features space is specified by the operator.

As stated above, all the ECGs extracted to a subset are sequentially combined into at least one cluster based on the similarity between their forms.

The clustering unit 3 checks, for the next analyzed ECG related to the extracted subset, the measure of similarity of its form with the forms of all ECGs in each of the already formed clusters A1 to A5.

If the analyzed ECG matches, by the predetermined form similarity measure, at least one of the ECGs of the already formed one cluster, the ECG is considered as corresponding to this cluster and, therefore, falling into this cluster. If the ECG corresponds to more than one cluster, the previously formed clusters, to which the analyzed ECG corresponds, are merged into a single cluster, e.g. A3 is merged with A4. If the ECG does not correspond to any of the previously formed clusters, a new cluster A5 containing this single ECG is formed (Fig. 2).

Furthermore, the measure of similarity of forms between any ECG pairs is specified as the distance between their corresponding pairs of DECG points, calculated by formulas for calculating the distance between points in at least one selected space: Euclidean space, Riemann space, Lobachevsky space, Hilbert space.

In long-term ECGs, a finite interval of 1 to 5 minutes is chosen, and DECG is determined in this interval for further calculation of the similarity measure.

In long-term ECGs, more than one finite interval is chosen, and DECG is determined in these intervals for further calculation of the similarity measure, and, the path of the patient's condition variation is constructed based on the similarity measure during the procedure of recording the long-term ECGs.

The space of features is normalized by reducing the recorded ECGs to one fixed length using an interpolation method.

Then, the STANDARD generation unit 5 generates a respective STANDARD for each cluster. The unit 5 generates a respective STANDARD B1 to B4 for each cluster A1 to A4 formed by the clustering unit 3 by forming a set of DECG points corresponding to ECGs of the cluster, and calculates characteristics of the generated set of DECG. The characteristics include: coordinates of the center of gravity 01, 02, 03, 04 (Fig. 2) of the generated DECG set, radius of the sphere or center of the polygon at the center of gravity of the DECG set, which includes all DECGs of the set.

The generated STANDARDS can be stored in the ECG database.

For subsequent assessment of the patient's condition, at least one STANDARD is assigned in accordance with the examination purpose. STANDARD is assigned by the STANDARD assignment unit 6 on doctor's request in accordance with the examination purpose for subsequent assessment of the patient's condition relative to the condition corresponding to the doctorassigned at least one STANDARD from the set of formed STANDARDs.

Variation in the observed patient's condition over time is evaluated by temporal variations in distances between each of the newly received DECGs in the patient's DECG series and the assigned at least one STANDARD.

Therefore, the assigned STANDARD corresponds to the condition indicated in the objective of examination.

Optionally, the stored STANDARD can be received from the ECG database.

Then, the observed patient ECG series is fed from the ECG database to the patient's condition assessment unit 7, where the variation in the observed patient's condition over time is evaluated by temporal variation in the distance between each of the newly received DECGs in the patient's DECG series and the assigned at least one STANDARD. Since the STANDARD is formed, the doctor or patient can compare one patient's ECG series received from the database with the at least one STANDARD.

The doctor's request is entered electronically by conventional methods.

It is important for the treating doctor and the patient to know the prognosis, for example, how the medication dosage must be adjusted depending on the observed patient's condition and how the patient's condition will change in future if the medication dosage is maintained, or whether a change in the dosage is required.

The present medical system provides the prognosis by the observed patient's condition prognosis unit 8, which predicts the most probable future condition of the patient on the basis of "observation history" comprising recorded temporal variations in past conditions of the observed patient relative to the assigned at least one STANDARD.

The observed patient's condition is predicted for future periods of time, starting from the last instant of recording the patient's ECG, based on the type of variation in the observed patient ECG path relative to the center of gravity of the assigned at least one STANDARD.

The medical system provides imaging of the patient's condition as the dynamics of variation in the distance of the patient's newly received ECGs at a displacement of geometric paths between the assigned STANDARDS.

The ECG series is recorded according to a predetermined examination method providing for recording ECGs at predetermined instants and/or upon occurrence of predetermined events related to patient's health status, patient's daily routine, medication intake or medical procedures.

The "with/without pathology" feature includes the presence or absence of pathology in the patient's diagnosis according to the International Classification of Diagnoses (ICD), confirmed by the doctor.

Optionally, electrocardiograms from the set consisting of Einthoven electrocardiogram, pulse wave cardiogram (photoplethysmogram), oxyhemogram, respiration card, echocardiogram, seismocardiogram and cardiovascular system signals, synchronized with ECGs can be used.

### Example

Fig. 3 shows schematically a variation in the patient's condition as the dynamics of variation in the distances of the newly received patient ECGs at a displacement of geometric paths between assigned STANDARDS.

The left side of Fig. 3 shows, by a vertical line, a previously received STANDARD of cardiograms belonging to young healthy individuals under 30 years of age who do not take any medications, and the right side shows a line for a previously received STANDARD of cardiograms belonging to elderly individuals over 60 years of age who take cardiac medications.

A patient takes his first-lead electrocardiogram several times a day (e.g. five times) using the cell phone application, and transfers data from the cell phone over the mobile operator's Internet to the database. From the database, the cardiograms are sequentially entered into the remote monitoring medical system at the request of the doctor who monitors and analyzes the patient's condition.

Upon receipt, the medical system processes each cardiogram, i.e. assigns it to a target group of patients, performs clustering, determines its similarity measure, forms a new STANDARD if necessary, and inputs in accordance with the STANDARD assigned by the doctor into the patient condition assessment unit, where the last received ECG is compared with the STANDARD. The comparison result is transferred to the unit for predicting the observed patient's condition by electrocardiograms (ECGs) series, which outputs a prognosis of the patient's future condition based on "observation history" comprising recorded variations in past conditions of the observed patient over time relative to the assigned at least one STANDARD.

The prognosis result enters the imaging unit, which displays the patient's condition as the dynamics of variations in the distance of the newly received patient's ECGs at a displacement of geometric paths between the assigned STANDARDS (Fig. 3).

### Industrial Applicability

The present computer-assisted method for monitoring, analysis and prognosis of the patient's condition by series of at least first-lead electrocardiograms (ECGs) and other cardiologic signals synchronized with the ECG provides an effective diagnosis of patients outside the healthcare center when determining the patient's condition and renders aid to both the patient in express assessment of his current condition and to the clinician in monitoring the course of treatment of the patient outside the healthcare center, including the assessment of cardiotoxicity in the application of medications, not only cardiac ones.

## Claims

1. A medical system for remote monitoring, analysis and prognosis of the patient's condition by series of at least first-lead electrocardiograms (ECGs) recorded for each patient from a plurality of observed patients, comprising:
a unit (1) for gathering in a database of said medical system for each of the observed patients a series of his electrocardiograms (ECGs) marked with patient identifiers, generated by adding each newly recorded patient's ECG marked with said identifiers to the patient's ECG series gathered previously throughout the entire observation time;
a unit (2) for extracting on doctor's request from the gathered database an ECG subset of a target group of patients, which are combined in said system into the target group in accordance with restrictions specified in the doctor's request and at least one of said identifiers in accordance with the examination purpose;
a unit (3) for clustering the extracted ECG subset, which sequentially combines, for at least one target group of patients combined by at least one feature, all the ECGs extracted to the subset to at least one cluster based on the feature of similarity of their forms using a clustering method;
a unit (4) for determining an ECG form similarity measure in the space of ECG form features, which associates each ECG from the database of the medical system with a discretized ECG (DECG), which is a point having coordinates in at least one space of ECG form features, chosen by the system operator, wherein coordinates of the point in said space of ECG form features carry information about the original ECG form, and the ECG form similarity measure between any ECG pairs is specified as the distance between their corresponding pairs of DECG points in this space;
a unit (5) for generating STANDARDS, which generates, for each cluster formed by the unit for clustering (3), a corresponding STANDARD by forming a set of DECG points corresponding to the cluster ECGs, and calculates characteristics of the formed DECG set, including: coordinates of the center of gravity of the formed DECG set, radius of the sphere or center of the polygon at the center of gravity of the DECG set, including all the DECG sets;
a unit (6) for assigning, on doctor's request, at least one STANDARD in accordance with the examination purpose from a plurality of generated STANDARDS for subsequent assessment of the patient's condition relative to the condition corresponding to the at least one STANDARD assigned by the doctor;
a unit (7) for assessing the patient's condition by the patient's ECG series, which assesses variation in the observed patient's condition over time by temporal variation in the distances between each newly received DECGs in the patient's DECG series and the assigned at least one STANDARD;
a unit (8) for predicting the observed patient's condition, which predicts the most probable future condition of the patient on the basis of "history of observations" comprising recorded variations of past observed patient's conditions over time relative to the assigned at least one STANDARD;
a unit (9) for imaging the patient's condition, which displays the dynamics of variation in distances of the patient's newly received ECGs as a displacement of cardiogram geometric paths between the assigned STANDARDS.

2. The medical system according to claim 1, wherein the unit (8) for predicting the observed patient's condition outputs a prognosis of the observed patient's condition in future periods of time, starting from the last instant of recording the patient's ECG, based on the type of variation in the observed patient's ECG path relative to the center of gravity of the assigned at least one STANDARD.

3. The medical system according to claim 1, wherein the patient identifiers contain two sets of features, a first set of features defining the patient's personal data code, and a second set of features comprising parameters of the outpatient's card selected from the group consisting of features of gender, age, diagnosis, social status, blood type, profession, belonging to social groups, education, genotype, with/without pathology, taking/not taking medications.

4. The medical system according to claim 1, wherein the form similarity measure between any ECG pairs is specified as the distance between their corresponding pairs of DECG points, calculated by formulas for calculating the distance between points in at least one selected space: Euclidean space, Riemannian space, Lobachevsky space, Hilbert space.

5. The medical system according to claim 1, wherein the clustering method is selected from the group consisting of: K-means, K-medians, C-means, EM, FOREL, Kohonen neural network, graph methods, including single link methods, complete link methods, Ward method, average link methods, weighted average link methods.

6. The medical system according to claim 1, wherein said clustering unit (3) checks, for the next analyzed ECG related to the extracted subset, the measure of similarity of its form with forms of all ECGs in each of the already formed clusters, and
if the analyzed ECG according to the predetermined form similarity measure matches at least one of the ECGs of one already formed cluster, then the ECG corresponds to this cluster and falls into this cluster;
if the ECG corresponds to more than one cluster, the previously formed clusters, to which the analyzed ECG corresponds, are merged into a single cluster;
if the ECG does not correspond to any of the previously formed clusters, a new cluster containing this single ECG is formed.

7. The medical system according to claim 1, wherein in long-term ECGs a finite time interval from 1 to 5 minutes is selected, and DECG is determined in this time interval for further calculation of the similarity measure.

8. The medical system according to claim 1, wherein in long-term ECGs more than one finite interval is selected; DECG is determined in these time intervals for further calculation of the similarity measure, and a path of patient's condition variation during the ECG recording procedure is constructed based on the similarity measure.

9. The medical system according to claim 1, wherein the space of features is normalized by reducing the recorded ECGs to one fixed length using an interpolation method.

10. The medical system according to claim 1, wherein characteristics of the generated DECG set include: coordinates of the center of gravity of the generated DECG set, radius of the sphere or center of the polygon at the center of gravity of the DECG set, including all the DECG sets.

11. A computer-assisted method for remote monitoring, analysis and prognosis of the patient's condition using a medical system according to claim 1, comprising:
gathering, for each patient from a plurality of observed patients, a series of patient's at least first-lead electrocardiograms (ECGs) marked with patient's identifiers in a database of said medical system, generated by adding each newly recorded patient's ECG marked with said identifiers to the patient's ECG series gathered previously throughout the entire observation period;
extracting, on doctor's request, from the gathered database an ECG subset of a target group of patients, which are combined into a target group in accordance with restrictions specified in the doctor's request and at least one of said identifiers in accordance with the examination purpose;
clustering the extracted ECG subset for the at least one target group of patients combined by at least one feature, for which purpose all the ECGs extracted to the subset are sequentially combined into at least one cluster by the feature of similarity between their forms using one of clustering methods;
determining a similarity measure of ECG forms in the space of ECG form features, for which purpose each ECG from the medical system database is associated with a discretized ECG (DECG), which is a point having coordinates in at least one space of ECG form features chosen by the system operator, and coordinates of the point in said space of ECG form features carry information about the original ECG form, and the measure of ECG form similarity between any ECG pairs is specified as the distance between their corresponding pairs of DECG points in this space;
generating a respective STANDARD for each cluster by forming a set of DECG points corresponding to the cluster ECGs and calculating characteristics of the formed DECG set, including: coordinates of the center of gravity of the formed DECG set, radius of the sphere or center of the polygon at the center of gravity of the DECG set, including all DECGs sets;
assigning at least one STANDARD from a set of generated standards for subsequent assessment of the patient's condition in accordance with the examination purpose specified by the doctor and comparing the patient's condition with the condition corresponding to the assigned at least one STANDARD;
assessing variation of the observed patient's condition over time by temporal variation in distances between each of the newly received DECGs in the patient's DECG series and the assigned at least one STANDARD;
predicting the observed patient's condition based on "observation history" comprising recorded variations in the past observed patient's conditions over time relative to the assigned at least one STANDARD;
imaging the patient's condition as the dynamics of variation in the distances of the newly received patient ECGs at a displacement of geometric paths between the assigned STANDARDS.

12. The method according to claim 11, wherein the observed patient's condition is predicted for future time periods, starting from the last instant of recording the patient's ECG, based on the type of variation in the observed patient's ECG path relative to the center of gravity of the assigned at least one STANDARD.

13. The method according to claim 11, wherein the patient identifiers are selected from two sets of features, a first set of features defining the patient's personal data code, and a second set of features comprising parameters of the outpatient's card selected from the set consisting of features of gender, age, diagnosis, social status, blood type, profession, belonging to social groups, education, genotype, with/without pathology, taking/not taking medications.

14. The method according to claim 11, wherein said recording of the ECG series is carried out according to a predetermined examination method, which provides for recording ECGs at specified instants and/or upon the occurrence of predetermined events associated with the patient's health status, patient's daily routine, taking medications or medical procedures.

15. The method according to claim 11, wherein the form similarity measure between any ECG pairs is specified as the distance between their corresponding pairs of DECG points, calculated by formulas for calculating the distance between points in at least one space: Euclidean space, Riemannian space, Lobachevsky space, Hilbert space.

16. The method according to claim 11, wherein the clustering method is selected from the group consisting of: K-means, K-medians, C-means, EM, FOREL, Kohonen neural network, graph methods, including single link methods, complete link methods, Ward method, average link methods, weighted average link methods.

17. The method according to claim 11, wherein the next analyzed ECG related to the extracted subset is clustered, for which purpose the similarity measure of its form with forms of all ECGs in each of the already formed clusters is checked, and
if the analyzed ECG matches, by the predetermined form similarity measure, at least one of ECGs of the already formed one cluster, the ECG is considered as corresponding to this cluster and falling into this cluster;
if the ECG corresponds to more than one cluster, the previously formed clusters, to which the analyzed ECG corresponds, are merged into a single cluster;
if the ECG does not correspond to any of the previously formed clusters, a new cluster containing this single ECG is formed.

18. The method according to claim 11, wherein the "with/without pathology" feature includes the presence or absence of pathology in the patient's diagnosis according to the International Classification of Diagnoses (ICD), confirmed by the doctor.

19. The method according to claim 11, further comprising using electrocardiograms selected from the set consisting of Eindhoven electrocardiogram, pulse wave cardiogram (photoplethysmogram), oxyhemogram, respiration card, echocardiogram, seismocardiogram and cardiovascular system signals synchronized with ECG.

20. The method according to claim 11, wherein characteristics of the generated DECG set include: coordinates of the center of gravity of the generated DECG set, radius of the sphere or center of the polygon at the center of gravity of the DECG set, including all DECG sets.

## Patentansprüche

1. Medizinisches System zur Fernüberwachung, -analyse und -prognose des Patientenzustandes durch eine Reihe von mindestens Elektrokardiogrammen (EKGs) der ersten Ableitung, die für jeden Patienten aus einer Vielzahl von beobachteten Patienten aufgenommen werden, umfassend:
eine Einheit (1), um in einer Datenbank des medizinischen Systems für jeden der beobachteten Patienten eine Reihe seiner Elektrokardiogramme (EKGs) zu sammeln, die mit Patientenkennungen markiert sind, die durch Hinzufügen jedes neu aufgenommenen EKGs des Patienten, das mit den Kennungen markiert ist, zu der EKG-Reihe des Patienten erzeugt werden, die zuvor während der gesamten Beobachtungszeit gesammelt wurde;
eine Einheit (2), um auf Anforderung des Arztes aus der gesammelten Datenbank eine EKG-Teilmenge einer Zielgruppe von Patienten zu extrahieren, die in dem System in Übereinstimmung mit den in der Anforderung des Arztes spezifizierten Einschränkungen und mindestens einem der Identifikatoren in Übereinstimmung mit dem Untersuchungszweck zu der Zielgruppe zusammengefasst werden;
eine Einheit (3) zum Clustern der extrahierten EKG-Teilmenge, die sequentiell für mindestens eine Zielgruppe von Patienten, die durch mindestens ein Merkmal zusammengefasst sind, alle zu der Teilmenge extrahierten EKGs zu mindestens einem Cluster basierend auf dem Merkmal der Ähnlichkeit ihrer Formen unter Verwendung eines Clustering-Verfahrens zusammenfasst;
eine Einheit (4) zum Bestimmen eines EKG-Formähnlichkeitsmaßes in dem Raum von EKG-Form-Merkmalen, die jedes EKG aus der Datenbank des medizinischen Systems einem diskretisierten EKG (DECG) zuordnet, das ein Punkt mit Koordinaten in mindestens einem Raum von EKG-Form-Merkmalen ist, der von dem Systembediener ausgewählt wird, wobei Koordinaten des Punktes in dem Raum von EKG-Form-Merkmalen Informationen über die ursprüngliche EKG-Form tragen und das EKG-Formähnlichkeitsmaß zwischen beliebigen EKG-Paaren als der Abstand zwischen ihren entsprechenden Paaren von DECG-Punkten in diesem Raum spezifiziert ist;
eine Einheit (5) zum Erzeugen von STANDARDS, die für jeden durch die Einheit zum Clustern (3) gebildeten Cluster einen entsprechenden STANDARD erzeugt, indem sie einen Satz von DECG-Punkten bildet, die den Cluster-EKGs entsprechen, und die Merkmale des gebildeten DECG-Satzes berechnet, einschließlich: Koordinaten des Schwerpunkts des gebildeten DECG-Satzes, Radius der Kugel oder Mittelpunkt des Polygons im Schwerpunkt des DECG-Satzes, einschließlich aller DECG-Sätze;
eine Einheit (6), die auf Anforderung des Arztes aus einer Vielzahl von erzeugten STANDARDS mindestens einen STANDARD in Übereinstimmung mit dem Untersuchungszweck für die nachfolgende Bewertung des Zustands des Patienten in Bezug auf den Zustand entsprechend dem mindestens einen vom Arzt zugeordneten STANDARD zuordnet;
eine Einheit (7) zur Beurteilung des Patientenzustandes anhand der EKG-Serien des Patienten, die die Veränderung des beobachteten Patientenzustandes über die Zeit durch die zeitliche Veränderung der Abstände zwischen jedem neu empfangenen DECG in der DECG-Serie des Patienten und dem zugeordneten mindestens einen STANDARD beurteilt;
eine Einheit (8) zur Vorhersage des beobachteten Patientenzustandes, die den wahrscheinlichsten zukünftigen Zustand des Patienten auf der Grundlage einer "Beobachtungsgeschichte" vorhersagt, die aufgezeichnete Variationen vergangener beobachteter Patientenzustände im Laufe der Zeit relativ zu dem zugeordneten mindestens einen STANDARD umfasst;
eine Einheit (9) zur Abbildung des Patientenzustandes, die die Dynamik der Abstandsänderung der neu empfangenen EKGs des Patienten als Verschiebung der geometrischen Pfade des Kardiogramms zwischen den zugeordneten STANDARDS anzeigt.

2. Medizinisches System nach Anspruch 1, wobei die Einheit (8) zur Vorhersage des beobachteten Patientenzustandes eine Prognose des beobachteten Patientenzustandes in zukünftigen Zeiträumen, beginnend mit dem letzten Zeitpunkt der Aufnahme des Patienten-EKGs, basierend auf der Art der Variation im EKG-Verlauf des beobachteten Patienten relativ zum Schwerpunkt des zugeordneten mindestens einen STANDARDs ausgibt.

3. Medizinisches System nach Anspruch 1, wobei die Patientenidentifikatoren zwei Sätze von Merkmalen enthalten, einen ersten Satz von Merkmalen, der den persönlichen Datencode des Patienten definiert, und einen zweiten Satz von Merkmalen, der Parameter der Ambulanzkarte umfasst, die aus der Gruppe ausgewählt werden, die aus den Merkmalen Geschlecht, Alter, Diagnose, sozialer Status, Blutgruppe, Beruf, Zugehörigkeit zu sozialen Gruppen, Bildung, Genotyp, mit/ohne Pathologie, Einnahme/Nichteinnahme von Medikamenten besteht.

4. Medizinisches System nach Anspruch 1, wobei das Formähnlichkeitsmaß zwischen beliebigen EKG-Paaren als der Abstand zwischen ihren entsprechenden Paaren von DECG-Punkten spezifiziert ist, berechnet durch Formeln zur Berechnung des Abstands zwischen Punkten in mindestens einem ausgewählten Raum: Euklidischer Raum, Riemannscher Raum, Lobachevsky-Raum, Hilbert-Raum.

5. Medizinisches System nach Anspruch 1, wobei das Clustering-Verfahren ausgewählt ist aus der Gruppe bestehend aus: K-Mittel, K-Median, C-Mittel, EM, FOREL, neuronales Kohonen-Netzwerk, Graphenmethoden, einschließlich Einzel-Link-Methoden, vollständige Link-Methoden, Ward-Methode, durchschnittliche Link-Methoden, gewichtete durchschnittliche Link-Methoden.

6. Medizinisches System nach Anspruch 1, wobei die Clustering-Einheit (3) für das nächste analysierte EKG, das sich auf die extrahierte Teilmenge bezieht, das Maß der Ähnlichkeit seiner Form mit den Formen aller EKGs in jedem der bereits gebildeten Cluster prüft, und
wenn das analysierte EKG nach dem vorgegebenen Formähnlichkeitsmaß mit mindestens einem der EKGs eines bereits gebildeten Clusters übereinstimmt, dann entspricht das EKG diesem Cluster und fällt in dieses Cluster;
wenn das EKG mehr als einem Cluster entspricht, werden die zuvor gebildeten Cluster, denen das analysierte EKG entspricht, zu einem einzigen Cluster zusammengeführt;
wenn das EKG keinem der zuvor gebildeten Cluster entspricht, wird ein neuer Cluster gebildet, der dieses einzelne EKG enthält.

7. Medizinisches System nach Anspruch 1, wobei bei Langzeit-EKGs ein endliches Zeitintervall von 1 bis 5 Minuten gewählt wird und in diesem Zeitintervall DECG für die weitere Berechnung des Ähnlichkeitsmaßes ermittelt wird.

8. Medizinisches System nach Anspruch 1, wobei in Langzeit-EKGs mehr als ein endliches Intervall ausgewählt wird; DECG in diesen Zeitintervallen für die weitere Berechnung des Ähnlichkeitsmaßes bestimmt wird und ein Pfad der Zustandsänderung des Patienten während des EKG-Aufzeichnungsvorgangs basierend auf dem Ähnlichkeitsmaß konstruiert wird.

9. Medizinisches System nach Anspruch 1, wobei der Raum von Merkmalen durch Reduzierung der aufgezeichneten EKGs auf eine feste Länge unter Verwendung eines Interpolationsverfahrens normalisiert wird.

10. Medizinisches System nach Anspruch 1, wobei die Merkmale des erzeugten DECG-Satzes Folgendes umfassen: Koordinaten des Schwerpunkts des erzeugten DECG-Satzes, Radius der Kugel oder Mittelpunkt des Polygons im Schwerpunkt des DECG-Satzes, einschließlich aller DECG-Sätze.

11. Computergestütztes Verfahren zur Fernüberwachung, -analyse und -prognose des Patientenzustandes unter Verwendung eines medizinischen Systems nach Anspruch 1, umfassend:
Sammeln, für jeden Patienten aus einer Vielzahl von beobachteten Patienten, einer Serie von Elektrokardiogrammen (EKGs) des Patienten mit mindestens der ersten Ableitung, die mit Identifikatoren des Patienten in einer Datenbank des medizinischen Systems markiert sind, erzeugt durch Hinzufügen jedes neu aufgezeichneten EKGs des Patienten, das mit den Identifikatoren markiert ist, zu der EKG-Serie des Patienten, die zuvor während des gesamten Beobachtungszeitraums gesammelt wurde;
Extrahieren einer EKG-Teilmenge einer Zielgruppe von Patienten aus der gesammelten Datenbank auf Anforderung des Arztes, wobei die Patienten gemäß den in der Anforderung des Arztes angegebenen Einschränkungen und mindestens einer der Identifikatoren gemäß dem Untersuchungszweck zu einer Zielgruppe zusammengefasst werden;
Clustering der extrahierten EKG-Teilmenge für die mindestens eine Patientenzielgruppe, die durch mindestens ein Merkmal zusammengefasst ist, wozu alle EKGs, die zu der Teilmenge extrahiert wurden, nacheinander in mindestens einem Cluster durch das Merkmal der Ähnlichkeit zwischen ihren Formen unter Verwendung einer der Clustering-Methoden zusammengefasst werden;
Bestimmung eines Ähnlichkeitsmaßes von EKG-Formen im Raum von EKG-Form-Merkmalen, wozu jedem EKG aus der Datenbank des medizinischen Systems ein diskretisiertes EKG (DECG) zugeordnet wird, das ein Punkt mit Koordinaten in mindestens einem Raum von EKG-Form-Merkmalen ist, der vom Systembetreiber ausgewählt wird, und die Koordinaten des Punktes in diesem Raum von EKG-Form-Merkmalen Informationen über die ursprüngliche EKG-Form tragen, und das Maß der EKG-FormÄhnlichkeit zwischen beliebigen EKG-Paaren als der Abstand zwischen ihren entsprechenden Paaren von DECG-Punkten in diesem Raum angegeben wird;
Erzeugen eines jeweiligen STANDARDs für jeden Cluster durch Bilden eines Satzes von DECG-Punkten, die den Cluster-EKGs entsprechen, und Berechnen von Merkmalen des gebildeten DECG-Satzes, einschließlich: Koordinaten des Schwerpunkts des gebildeten DECG-Satzes, Radius der Kugel oder Mittelpunkt des Polygons im Schwerpunkt des DECG-Satzes, einschließlich aller DECG-Sätze;
Zuordnung von mindestens einem STANDARD aus einem Satz generierter Standards für die spätere Beurteilung des Zustands des Patienten entsprechend dem vom Arzt vorgegebenen Untersuchungszweck und Vergleich des Zustands des Patienten mit dem Zustand, der dem zugeordneten mindestens einen STANDARD entspricht;
Bewertung der Veränderung des beobachteten Patientenzustandes im Laufe der Zeit durch zeitliche Veränderung der Abstände zwischen jedem der neu empfangenen DECGs in der DECG-Serie des Patienten und dem zugeordneten mindestens einen STANDARD;
Vorhersage des beobachteten Patientenzustands basierend auf einer "Beobachtungshistorie", die aufgezeichnete Variationen des vergangenen beobachteten Patientenzustands im Laufe der Zeit im Verhältnis zu dem zugeordneten mindestens einen STANDARD umfasst;
Abbildung des Patientenzustandes als Dynamik der Veränderung der Abstände der neu empfangenen Patienten-EKGs bei einer Verschiebung der geometrischen Pfade zwischen den zugeordneten STANDARDS.

12. Verfahren nach Anspruch 11, wobei der Zustand des beobachteten Patienten für künftige Zeiträume, beginnend mit dem letzten Zeitpunkt der Aufzeichnung des EKGs des Patienten, basierend auf der Art der Veränderung des EKG-Verlaufs des beobachteten Patienten relativ zum Schwerpunkt des zugeordneten mindestens einen STANDARDs vorhergesagt wird.

13. Verfahren nach Anspruch 11, wobei die Patientenidentifikatoren aus zwei Merkmalsgruppen ausgewählt werden, einer ersten Merkmalsgruppe, die den persönlichen Datencode des Patienten definiert, und einer zweiten Merkmalsgruppe, die Parameter der Ambulanzkarte umfasst, die aus der Gruppe ausgewählt werden, die aus folgenden Merkmalen besteht: Geschlecht, Alter, Diagnose, sozialer Status, Blutgruppe, Beruf, Zugehörigkeit zu sozialen Gruppen, Bildung, Genotyp, mit/ohne Pathologie, Einnahme/Nichteinnahme von Medikamenten.

14. Verfahren nach Anspruch 11, wobei die Aufzeichnung der EKG-Serien nach einem vorbestimmten Untersuchungsverfahren durchgeführt wird, das die Aufzeichnung von EKGs zu bestimmten Zeitpunkten und/oder beim Auftreten vorbestimmter Ereignisse vorsieht, die mit dem Gesundheitszustand des Patienten, dem Tagesablauf des Patienten, der Einnahme von Medikamenten oder medizinischen Verfahren zusammenhängen.

15. Verfahren nach Anspruch 11, wobei das Maß für die Formähnlichkeit zwischen beliebigen EKG-Paaren als der Abstand zwischen ihren entsprechenden Paaren von DECG-Punkten spezifiziert wird, der durch Formeln zur Berechnung des Abstands zwischen Punkten in mindestens einem Raum berechnet wird: Euklidischer Raum, Riemannscher Raum, Lobachevsky-Raum, Hilbert-Raum.

16. Verfahren nach Anspruch 11, wobei das Clustering-Verfahren ausgewählt ist aus der Gruppe bestehend aus: K-Mittel, K-Median, C-Mittel, EM, FOREL, neuronales Kohonen-Netzwerk, Graph-Methoden, einschließlich Einzelverbindungsmethoden, vollständige Verbindungsmethoden, Ward-Methode, durchschnittliche Verbindungsmethoden, gewichtete durchschnittliche Verbindungsmethoden.

17. Verfahren nach Anspruch 11, wobei das nächste analysierte EKG, das sich auf die extrahierte Teilmenge bezieht, geclustert wird, wozu das Ähnlichkeitsmaß seiner Form mit den Formen aller EKGs in jedem der bereits gebildeten Cluster geprüft wird, und
wenn das analysierte EKG durch das vorgegebene Formähnlichkeitsmaß mit mindestens einem der EKGs des bereits gebildeten Clusters übereinstimmt, wird das EKG als diesem Cluster zugehörig betrachtet und fällt in dieses Cluster;
wenn das EKG mehr als einem Cluster entspricht, werden die zuvor gebildeten Cluster, denen das analysierte EKG entspricht, zu einem einzigen Cluster zusammengeführt;
wenn das EKG keinem der zuvor gebildeten Cluster entspricht, wird ein neuer Cluster gebildet, der dieses einzelne EKG enthält.

18. Verfahren nach Anspruch 11, wobei das Merkmal "mit/ohne Pathologie" das Vorhandensein oder Fehlen einer Pathologie in der vom Arzt bestätigten Diagnose des Patienten gemäß der Internationalen Klassifikation der Diagnosen (ICD) umfasst.

19. Verfahren nach Anspruch 11, das weiter die Verwendung von Elektrokardiogrammen umfasst, die aus der Gruppe ausgewählt sind, die aus dem Eindhoven-Elektrokardiogramm, dem Pulswellenkardiogramm (Photoplethysmogramm), dem Oxyhämogramm, der Atmungskarte, dem Echokardiogramm, dem Seismokardiogramm und den mit dem EKG synchronisierten Signalen des Herz-KreislaufSystems besteht.

20. Verfahren nach Anspruch 11, wobei die Merkmale des erzeugten DECG-Satzes Folgendes beinhalten: Koordinaten des Schwerpunkts des erzeugten DECG-Satzes, Radius der Kugel oder Mittelpunkt des Polygons im Schwerpunkt des DECG-Satzes, einschließlich aller DECG-Sätze.

## Revendications

1. Système médical pour une surveillance, une analyse et un pronostic à distance de l'état d'un patient par une série d'au moins des électrocardiogrammes (ECG) de première dérivation enregistrés pour chaque patient à partir d'une pluralité de patients observés, comprenant :
une unité (1) pour collecter dans une base de données dudit système médical pour chacun des patients observés une série de ses électrocardiogrammes (ECG) marqués avec des identifiants de patient, générés en ajoutant chaque ECG de patient nouvellement enregistré marqué avec lesdits identifiants à la série d'ECG du patient collectée au préalable pendant toute la durée de l'observation ;
une unité (2) pour extraire à la demande du médecin à partir de la base de données collectée un sous-ensemble d'ECG d'un groupe cible de patients, qui sont combinés dans ledit système en le groupe cible conformément aux restrictions spécifiées dans la demande du médecin et au moins un desdits identifiants conformément au but d'examen ;
une unité (3) de regroupement du sous-ensemble d'ECG extrait, qui combine séquentiellement, pour au moins un groupe cible de patients combinés par au moins une caractéristique, tous les ECG extraits du sous-ensemble en au moins un regroupement sur la base de la caractéristique de similitude de leurs formes en utilisant un procédé de regroupement ;
une unité (4) pour déterminer une mesure de similitude de forme d'ECG dans l'espace des caractéristiques de forme d'ECG, qui associe chaque ECG de la base de données du système médical à un ECG discrétisé (DECG), qui est un point présentant des coordonnées dans au moins un espace de caractéristiques de forme d'ECG, choisi par l'opérateur du système, dans lequel les coordonnées du point dans ledit espace de caractéristiques de forme d'ECG portent des informations concernant la forme d'ECG d'origine, et la mesure de similitude de forme d'ECG entre de quelconques paires d'ECG est spécifiée comme la distance entre leurs paires correspondantes de points de DECG dans cet espace ;
une unité (5) de génération de NORMES, qui génère, pour chaque groupe formé par l'unité de regroupement (3), une NORME correspondante en formant un ensemble de points de DECG correspondant aux ECG de groupe, et calcule des caractéristiques de l'ensemble de DECG formé, y compris : des coordonnées du centre de gravité de l'ensemble de DECG formé, un rayon de la sphère ou du centre du polygone au niveau du centre de gravité de l'ensemble de DECG, y compris tous les ensembles de DECG ;
une unité (6) pour attribuer, à la demande du médecin, au moins une NORME conformément au but de l'examen parmi une pluralité de NORMES générées pour une évaluation ultérieure de l'état du patient par rapport à l'état correspondant à ladite au moins une NORME attribuée par le médecin ;
une unité (7) pour évaluer l'état du patient par la série d'ECG du patient, qui évalue la variation de l'état du patient observé dans le temps par une variation temporelle des distances entre chaque DECG nouvellement reçu dans la série de DECG du patient et la au moins une NORME attribuée ;
une unité (8) pour prédire l'état du patient observé, qui prédit l'état futur le plus probable du patient sur la base d'un « historique des observations » comprenant des variations enregistrées des états du patient observé passés au fil du temps par rapport à la au moins une NORME attribuée ;
une unité (9) d'imagerie de l'état du patient, qui affiche la dynamique de variation des distances des ECG nouvellement reçus du patient sous la forme d'un déplacement de trajectoires géométriques de cardiogramme entre les NORMES attribuées.

2. Système médical selon la revendication 1, dans lequel l'unité (8) pour prédire l'état du patient observé délivre en sortie un pronostic de l'état du patient observé dans des périodes de temps futures, en commençant par le dernier instant d'enregistrement de l'ECG du patient, sur la base du type de variation de la trajectoire d'ECG du patient observé par rapport au centre de gravité de la au moins une NORME attribuée.

3. Système médical selon la revendication 1, dans lequel les identifiants de patient contiennent deux ensembles de caractéristiques, un premier ensemble de caractéristiques définissant le code de données personnelles du patient, et un second ensemble de caractéristiques comprenant des paramètres de la carte de patient externe sélectionnés dans le groupe constitué de caractéristiques de sexe, âge, diagnostic, statut social, groupe sanguin, profession, appartenance à des groupes sociaux, éducation, génotype, avec/sans pathologie, prise/non prise de médicaments.

4. Système médical selon la revendication 1, dans lequel la mesure de similitude de forme entre de quelconques paires d'ECG est spécifiée comme la distance entre leurs paires correspondantes de points de DECG, calculée par des formules pour calculer la distance entre les points dans au moins un espace sélectionné : espace euclidien, espace Riemannien, espace de Lobachevsky, espace de Hilbert.

5. Système médical selon la revendication 1, dans lequel le procédé de regroupement est sélectionné dans le groupe consistant en : K-moyennes, K-médianes, C-moyennes, EM, FOREL, réseau neuronal de Kohonen, procédés de graphe, y compris procédés de liaison unique, procédés de liaison complète, procédé de Ward, procédés de liaison moyenne, procédés de liaison moyenne pondérée.

6. Système médical selon la revendication 1, dans lequel ladite unité de regroupement (3) vérifie, pour l'ECG analysé suivant lié au sous-ensemble extrait, la mesure de similitude de sa forme avec les formes de tous les ECG dans chacun des groupes déjà formés, et
si l'ECG analysé selon la mesure de similitude de forme prédéterminée correspond à au moins un des ECG d'un groupe déjà formé, alors l'ECG correspond à ce groupe et tombe dans ce groupe ;
si l'ECG correspond à plus d'un groupe, les groupes précédemment formés, auxquels correspond l'ECG analysé, sont fusionnés en un groupe unique ;
si l'ECG ne correspond à aucun des groupes précédemment formés, un nouveau groupe contenant cet ECG unique est formé.

7. Système médical selon la revendication 1, dans lequel, dans les ECG à long terme, un intervalle de temps fini de 1 à 5 minutes est sélectionné, et le DECG est déterminé dans cet intervalle de temps pour un calcul supplémentaire de la mesure de similitude.

8. Système médical selon la revendication 1, dans lequel, dans les ECG à long terme, plus d'un intervalle fini est sélectionné ; le DECG est déterminé dans ces intervalles de temps pour un calcul supplémentaire de la mesure de similitude, et un trajet de variation de l'état du patient pendant la procédure d'enregistrement d'ECG est construit sur la base de la mesure de similitude.

9. Système médical selon la revendication 1, dans lequel l'espace de caractéristiques est normalisé en réduisant les ECG enregistrés à une longueur fixe en utilisant un procédé d'interpolation.

10. Système médical selon la revendication 1, dans lequel des caractéristiques de l'ensemble de DECG généré incluent : des coordonnées du centre de gravité de l'ensemble de DECG généré, un rayon de la sphère ou du centre du polygone au niveau du centre de gravité de l'ensemble de DECG, y compris tous les ensembles de DECG.

11. Procédé assisté par ordinateur pour une surveillance, une analyse et un pronostic à distance de l'état du patient en utilisant un système médical selon la revendication 1, comprenant les étapes consistant à :
collecter, pour chaque patient parmi une pluralité de patients observés, une série d'au moins des électrocardiogrammes (ECG) de première dérivation du patient marqués avec des identifiants de patient dans une base de données dudit système médical, générés en ajoutant chaque ECG de patient nouvellement enregistré marqué avec lesdits identifiants à la série d'ECG du patient collectée au préalable pendant toute la période d'observation ;
extraire, à la demande du médecin, de la base de données collectée un sous-ensemble ECG d'un groupe cible de patients, qui sont combinés en un groupe cible conformément aux restrictions spécifiées dans la demande du médecin et au moins un desdits identifiants conformément à l'objectif de l'examen ;
regrouper le sous-ensemble d'ECG extrait pour le au moins un groupe cible de patients combinés par au moins une caractéristique, but pour lequel tous les ECG extraits du sous-ensemble sont combinés séquentiellement en au moins un groupe par la caractéristique de similitude entre leurs formes en utilisant l'un des procédés de regroupement ;
déterminer une mesure de similitude de formes d'ECG dans l'espace de caractéristiques de forme d'ECG, but pour lequel chaque ECG provenant de la base de données du système médical est associé à un ECG discrétisé (DECG), qui est un point présentant des coordonnées dans au moins un espace de caractéristiques de forme d'ECG choisi par l'opérateur du système, et les coordonnées du point dans ledit espace de caractéristiques de forme d'ECG portent des informations sur la forme d'ECG d'origine, et la mesure de la similitude de forme d'ECG entre de quelconques paires d'ECG est spécifiée comme la distance entre leurs paires correspondantes de points de DECG dans cet espace ;
générer une NORME respective pour chaque groupe en formant un ensemble de points de DECG correspondant aux ECG de groupe et en calculant de caractéristiques de l'ensemble de DECG formé, y compris : des coordonnées du centre de gravité de l'ensemble de DECG formé, un rayon de la sphère ou du centre du polygone au niveau du centre de gravité de l'ensemble de DECG, y compris tous les ensembles de DECG ;
attribuer au moins une NORME parmi un ensemble de normes générées pour une évaluation ultérieure de l'état du patient conformément au but d'examen spécifié par le médecin et comparer l'état du patient avec l'état correspondant à la au moins une NORME attribuée ;
évaluer une variation de l'état du patient observé dans le temps par la variation temporelle de distances entre chacun des DECG nouvellement reçus dans la série de DECG du patient et la au moins une NORME attribuée ;
prédire l'état du patient observé sur la base d'un « historique des observations » comprenant des variations enregistrées de l'état du patient passé observé dans le temps par rapport à la au moins une NORME attribuée ;
imager l'état du patient sous la forme de la dynamique de variation des distances des ECG de patient nouvellement reçus lors d'un déplacement de trajectoires géométriques entre les NORMES attribuées.

12. Procédé selon la revendication 11, dans lequel l'état du patient observé est prédit pour des périodes de temps futures, à partir du dernier instant d'enregistrement de l'ECG du patient, sur la base du type de variation de la trajectoire d'ECG du patient observé par rapport au centre de gravité de la au moins une NORME attribuée.

13. Procédé selon la revendication 11, dans lequel les identifiants de patient sont sélectionnés parmi deux ensembles de caractéristiques, un premier ensemble de caractéristiques définissant le code de données personnelles du patient, et un second ensemble de caractéristiques comprenant des paramètres de la carte de patient externe sélectionnés parmi l'ensemble constitué de caractéristiques de sexe, âge, diagnostic, statut social, groupe sanguin, profession, appartenance à des groupes sociaux, éducation, génotype, avec/sans pathologie, prise/non prise de médicaments.

14. Procédé selon la revendication 11, dans lequel ledit enregistrement de la série d'ECG est effectué selon un procédé d'examen prédéterminé, qui prévoit l'enregistrement d'ECG à des instants spécifiés et/ou lors de la survenue d'événements prédéterminés associés à l'état de santé du patient, à la routine quotidienne du patient, à une prise de médicaments ou des procédures médicales.

15. Procédé selon la revendication 11, dans lequel la mesure de similitude de forme entre de quelconques paires d'ECG est spécifiée comme la distance entre leurs paires correspondantes de points de DECG, calculée par des formules pour calculer la distance entre les points dans au moins un espace : espace euclidien, espace Riemannien, espace de Lobachevsky, espace de Hilbert.

16. Procédé selon la revendication 11, dans lequel le procédé de regroupement est sélectionné dans le groupe consistant en : K-moyennes, K-médianes, C-moyennes, EM, FOREL, réseau neuronal de Kohonen, des procédés de graphe, y compris des procédés de liaison unique, des procédés de liaison complète, un procédé de Ward, des procédés de liaison moyenne, des procédés de liaison moyenne pondérée.

17. Procédé selon la revendication 11, dans lequel l'ECG analysé suivant lié au sous-ensemble extrait est regroupé, but pour lequel la mesure de similitude de sa forme avec les formes de tous les ECG dans chacun des groupes déjà formés est vérifiée, et
si l'ECG analysé correspond, par la mesure de similitude de forme prédéterminée, à au moins un des ECG du groupe déjà formé, l'ECG est considéré comme correspondant à ce groupe et tombant dans ce groupe ;
si l'ECG correspond à plus d'un groupe, les groupes précédemment formés, auxquels correspond l'ECG analysé, sont fusionnés en un groupe unique ;
si l'ECG ne correspond à aucun des groupes précédemment formés, un nouveau groupe contenant cet ECG unique est formé.

18. Procédé selon la revendication 11, dans lequel la caractéristique « avec/sans pathologie » inclut la présence ou l'absence de pathologie dans le diagnostic du patient selon la Classification internationale des diagnostics (CIM), confirmée par le médecin.

19. Procédé selon la revendication 11, comprenant en outre l'utilisation d'électrocardiogrammes sélectionnés dans l'ensemble constitué par électrocardiogramme d'Eindhoven, cardiogramme à ondes pulsées (photopléthysmogramme), oxyhémogramme, carte de respiration, échocardiogramme, sismocardiogramme et signaux de système cardiovasculaire synchronisés avec l'ECG.

20. Procédé selon la revendication 11, dans lequel les caractéristiques de l'ensemble de DECG généré incluent : des coordonnées du centre de gravité de l'ensemble de DECG généré, un rayon de la sphère ou du centre du polygone au niveau du centre de gravité de l'ensemble de DECG, y compris tous les ensembles de DECG.
